(19) Europäisches Patentamt
European Patent Office
Office européen des brevets

(11) Publication number: 0 103 948
A1

# (12) EUROPEAN PATENT APPLICATION

(21) Application number: 83303887.0

(22) Date of filing: 04.07.83

(51) Int. Cl.³: C 07 C 9/04
C 10 G 45/58, C 10 G 69/06
C 07 C 4/00, C 07 C 5/10

(30) Priority: 23.08.82 GB 8224146

(43) Date of publication of application:
28.03.84 Bulletin 84/13

(84) Designated Contracting States:
BE DE FR IT NL

(71) Applicant: British Gas Corporation
Rivermill House 152 Grosvenor Road
London SW1V 3JL(GB)

(72) Inventor: Ensell, Robert Leslie
67 Coppice Walk
Shirley Solihull West Midlands B90 6HZ(GB)

## (54) Producing methane rich gases.

(57) Polycyclic aromatic compounds present in reaction products obtained by the thermal hydrogenation of hydrocarbon liquids such as crude oils, may be catalytically hydrogenated under mild temperature conditions and high hydrogen partial pressures to produce saturated species but without ring fission. The saturated species may then be thermally hydrogenated to produce monocyclic aromatics and aliphatic fuel components such as ethane and methane.

The process may be employed as a modification of the process described in UK Patent Application Publication No. 2062000 wherein a subsidiary thermal hydrogenation reaction is replaced with a catalytic hydrogenation stage.

EP 0 103 948 A1

./...

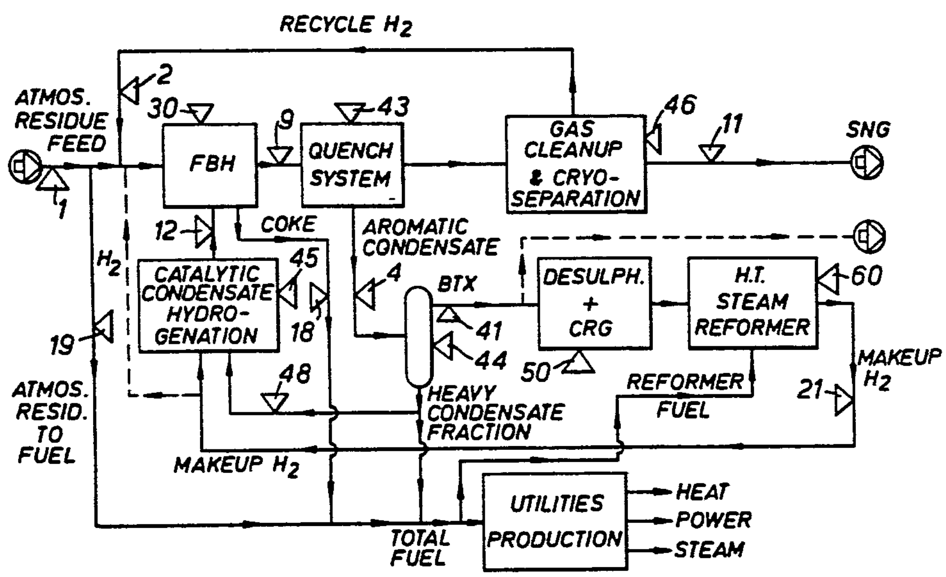

RECYCLE $H_2$
ATMOS. RESIDUE FEED
FBH
QUENCH SYSTEM
GAS CLEANUP & CRYO-SEPARATION
SNG
COKE
AROMATIC CONDENSATE
$H_2$
CATALYTIC CONDENSATE HYDRO-GENATION
BTX
DESULPH. + CRG
H.T. STEAM REFORMER
MAKEUP $H_2$
REFORMER FUEL
HEAVY CONDENSATE FRACTION
ATMOS. RESID. TO FUEL
MAKEUP $H_2$
UTILITIES PRODUCTION
HEAT
POWER
STEAM
TOTAL FUEL
1
2
30
9
43
46
11
12
45
18
4
41
44
50
60
21
19
48

This invention relates to the production of monocyclic and saturated polycyclic hydrocarbons. More particularly the invention is for an improvement in the production of gases which are useful as substitute natural gas (SNG) by the thermal hydrogenation of liquid hydrocarbons.

The non catalytic thermal hydrogenation of hydrocarbon liquids as a primary gasification process for the manufacture of SNG is well known. For example a primary gasification process is described in our UK PS 830960 in which an oil is vaporised in a stream of a hot hydrogen-containing gas and the mixture atomised into a recirculating fluidised bed of, for example, particles of a carbonaceous material. The oil deposited on the particles is thermally hydrogenated to form a product gas comprising methane and condensible polynuclear hydrocarbons which together with unreacted hydrogen are removed from the reactor bed. The gas is then treated to separate out the condensate and the hydrogen which can be reused. The disadvantage of this process is that condensate cannot be gasified readily.

Further research work on the treatment of condensate has been carried out and in our UK Patent Application Publication No. 2062000 there are *inter alia* described a number of process techniques for treating condensate to enable it to be converted to SNG or a usable precursor.

The techniques described include catalytic cracking to convert the polynuclear species to mononuclear ones which can then be subjected to catalytic steam reforming. Alternatively the condensate may first be subjected to a separation process to remove the indigenous mononuclear aromatic hydrocarbons. The residue or heavy condensate fraction is then subjected to a non-catalytic thermal hydrogenation process before being returned to the primary hydrogenation stage together with fresh feedstock. However, in order to achieve adequate ring fission and/or saturation, much higher temperatures than those employed in the primary hydrogenator are required. Published data indicates that temperatures in excess of 1000$^{o}$C are required to achieve 99% conversion of the multi-ring species. This inevitably requires the use of sophisticated materials to withstand the severe operating conditions. Moreover, the use of temperatures of this order does not favour maximising the concentration of methane and other desirable high calorific value components such as ethane, in the equilibrium composition.

We have found that the reactivity of the ring systems can be substantially increased by a catalytic hydrogenation stage in which the aromatic ring systems are partially saturated. This, surprisingly, enables substantial conversion of the multi ring compounds to compounds of a naphthenic type structure at temperatures similar to those

BAD ORIGINAL

employed in a primary gasification reactor such as described in UK 2062000. Thus products from the catalytic hydrogenator may be passed directly to a primary gasifier without the need for either heating or cooling.

In accordance with the present invention there is provided a process for the production of polynuclear saturated compounds which comprises subjecting a feedstock comprising predominantly unsaturated polynuclear hydrocarbons to catalytic hydrogenation at a temperature of not more than 500°C with a hydrogen-containing gas at a partial pressure of at least 100 bar, the ratio of hydrogen to feedstock being not less than 0.9Nm$^3$ ($H_2$) pr kg (feedstock), said catalyst being one which has substantially no propensity for cracking or for initiating and sustaining gasification reactions at said temperatures and pressures.

The present invention further provides a process for the production of methane containing gases which process comprises subjecting a feedstock comprising predominantly unsaturated polynuclear hydrocarbon compounds to catalytic hydrogenation at a temperature not greater than 500°C with a hydrogen containing gas at a partial pressure of at least 100 bar, the ratio of hydrogen to oil being not less than 0.9 Nm$^3$ hydrogn per kg of feedstock, said catalyst having substantially no propensity for cracking or for initiation and sustaining gasification reactions at said temperatures

and pressures, to produce a product comprising saturated polynuclear hydrocarbon compounds and thereafter subjecting said product to non-catalytic thermal hydrogenation at a temperature of not more than 950°C in the presence of a hydrogen-containing gas.

It is preferred to carry out the process of the invention at temperatures in the range of from 350°C to 450°C. At temperatures in excess of 500°C the equilibrium composition of the product gas will be significantly altered together with the onset of gasification reactions which due to their exothermic nature will raise the temperature of the product gas again affecting the equilibrium composition.

At hydrogen to feed ratios of less than 0.9 $Nm^3\ kg^{-1}$ coking reactions may occur, thus deactivating the catalyst. It is therefore preferred to employ ratios within the range 1.2 - 2.5 $Nm^3$ ($H_2$) $kg^{-1}$ (feedstock). Ratios in excess of 2.5 $Nm^3\ kg^{-1}$ do not onfer any additional benefits.

In order to obtain the necessary saturation without substantial ring cleavage it is essential to employ hydrogen partial pressures in excess of 100 bar. A preferred range is from 100 to 200 bar. All pressures stated herein are expressed as gauge pressures.

In the process of the present invention unsaturated compounds are catalytically hydrogenated without ring fission to form more saturated species. For example typical reactions are

(i)

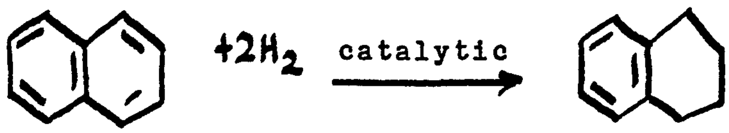


(ii)

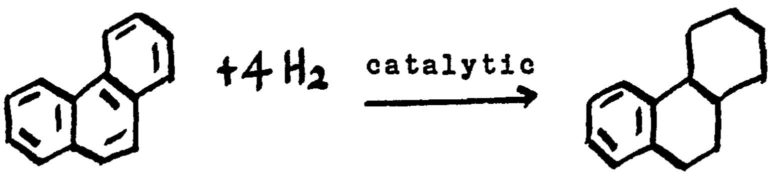


On further treatment by non-catalytic hydrogenation, ring fission occurs to produce mononuclear aromatic species and aliphatic compounds. Thus:

(iii)

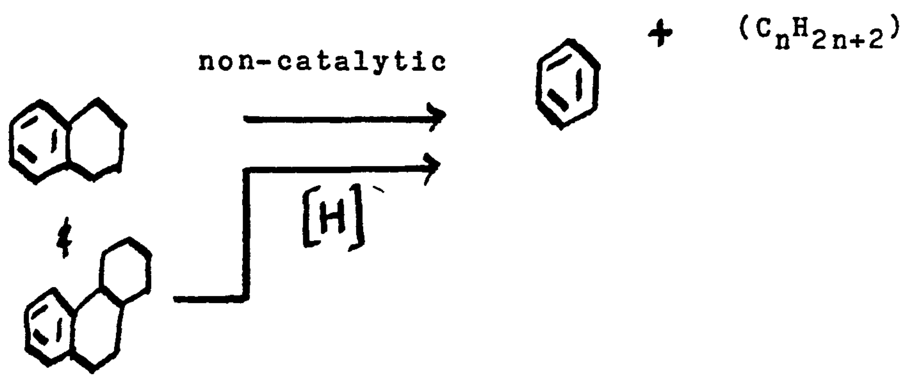


methane and ethane being the predominant aliphatic species.

Catalysts for use in the process of the invention and having desired properties are commercially available and include sulphided forms of nickel and tungsten, nickel and molybdenum and cobalt and molybdeum, all supported on

alumina. Examples of suitable commercial catalysts are Harshaw NiW-216 and Ni-4301, nickel-tungsten catalysts and Cyanamid HDN-30, a nickel-molybdeum catalyst.

It is essential that the hydrogenation catalysts have little propensity for cracking or for gasifying since these reactions are undesirable due to their high exothermicity, and consequent high hydrogen consumption.

The process of the present invention may be integrated into the process described in UK 2062000. For example, the embodiment described with reference to Figure 5 of UK 2062000 may be modified by replacing the recycle condensate hydrogenator 45 with a catalytic hydrogenator as shown in the accompanying drawing.

Referring to the drawing which is a block schematic flow diagram of an overall process for the production of SNG from an atmospheric residue feestock derived from a crude oil feedstock, the feedstock is taken from storage (not shown) through line 1 and passed into a thermal hydrogenator 30 together with hydrogen-containing gas from line 2. An additional feedstock comprising predominantly saturated polynuclear hydrocarbons is fed into the hydrogenator through line 12. Gaseous products from the hydrogenator are withdrawn through line 9. The thermal hydrogenator 30 may be a fluidised bed hydrogenator (FBH) constructed to operate in the manner described, for

example, in UK Patent No. 830960, and any solid products are withdrawn via line 18 for use in service utilities.

The gaseous product for line 9, comprising methane, other alkyl hydrocarbons, mono and polynuclear aromatics, carbon oxides and unreacted hydrogen are subjected to a quench separation to remove condensible materials, and the gas phase is further treated in the clean up and hydrogen separation stage in the cryogenic unit 46. The product fuel gas component from 46 is taken out via line 11, and the hydrogen recycled to unit 30 via line 2.

The aromatic condensate derived from the quench 43 is subjected to further separation in unit 44 to form a lighter fraction (41) comprising mono-nuclear aromatics such as benzene, toluene and xylene (BTX) and a heavy condensate fraction (48) The BTX fraction (41) is subjected to
catalytic gasification and high temperature steam reforming in units 50 and 60 to produce hydrogen which is admixed via line 21 with the heavy condensate fraction (48) in catalytic hydrogenation unit 45. In the hydrogenator 45 the unsaturated polynuclear hydrocarbons of the heavy condensate fraction are saturated to form a product which is removed via line 12 and added as additional feedstock to the primary hydrogenator 30.

In this way the present invention may be used to modify the process described and claimed in our UK Application Publication No. 2062000. Accordingly, there is further provided an integrated process for the production of substitute natural gas or precursors therefor comprising thermally hydrogenating a hydrocarbon oil in the presence of a hydrogen containing gas at a temperature of not more than 950°C, separating from the reaction product a methane rich gas, unreacted hydrogen-containing gas and a condensible material comprising poly and monocyclic compounds, further separating the condensible material into mono and polycyclic components, reacting the monocyclic components over a reforming catalyst to produce a hydrogen containing product, reacting a mixture of said hydrogen-containing product and said polycyclic component in a ratio of not less than 0.9 $Nm^3$ of hydrogen containing gas per kg of polycyclic component in the presence of a hydrogenating catalyst having substantially no propensity for cracking or for initiating and sustaining gasification reactions at temperatures of not more than 500°C and at a hydrogen partial pressure of at least 100 bar thereby to form a reaction product comprising saturated polynuclear hydrocarbons and thereafter thermally hydrogenating said saturated polynuclear hydrocarbon reaction product together with said hydrocarbon oil.

The invention and the advantages accruing thereto will be illustrated by the following Example.

A series of tests were conducted in which an aromatic feedstock boiling in the range 150° - 470°C was subjected to thermal hydrogenation after a catalytic hydrogenation in accordance with the invention and as a comparison without prior catalytic hydrogenation.

The feedstock is obtained by subjecting a Kuwait Atmospheric Residual Oil to thermal hydrogenation in fluidised bed reactor (30) in accordance with the procedure described in UK 2062000A and under the following conditions:

| | | | |
|---|---|---|---|
| Pressure: | | 50 bar (gauge) | |
| Inlet Temperature | - | Hydrogen | 360°C |
| | - | Feedstock | 300°C |
| Hydrogen/Feedstock ratio | | 2.7/1 | $Nm^3/Kg$ |
| Outlet Temperature | | | 750°C |

The product gas was subjected to quenching in unit 43 to remove the aromatic condensate (4) which is subjected to distillation in the fractionation unit 44. Three fractions are obtained:

| | | |
|---|---|---|
| < 159°C | - | 38% by weight |
| 150 - 470°C | - | 42% by weight |
| > 470°C | - | 20% by weight |

The < 150°C fraction (41) consisting largely of BTX will go to the reformers 50 and 60 whilst the > 470°C fraction is used for fuel and for utilities production. The middle fraction (48) is used as the feedstock.

A sample of this 150° - 470°C fraction feedstock (48) having the composition shown in column A of the Table was catalytically hydrogenated under the following conditions:

| | |
|---|---|
| Pressure bar | 100 |
| Temperature °C | 450 |
| Space Velocity $hr^{-1}$ | 1.0 |
| $H_2$/Feed ratio $Nm^3/Kg$ | 1.25/1 |
| Catalyst | Cyanamid HDN - 30 |

to give a product of a composition given in column B. This product was then thermally hydrogenated under the following conditions:

| | |
|---|---|
| Pressure bar | 50 |
| Temperature °C | 759 |
| Cat Prod/$H_2$ ratio, g/litre | 0.631/1, |

to give a product of a composition given in column C.

In a comparative experiment a further sample of the

original feedstock was directly thermally hydrogenated, without prior catalytic hydrogenation under the following conditions:

| | |
|---|---|
| Pressure bar | 49 |
| Temperature °C | 766 |
| Feedstock/$H_2$ratio,g/litre | 0.742 |

to give a product of the composition given in column D.

| Component | A | B | C | D |
|---|---|---|---|---|
| Benzene | 2.1 | 1.9 | 34.3 | 11.1 |
| Toluene < naphthalene | 1.38 | 6.71 | 3.02 | 2.09 |
| Naphthalene | 20.3 | 5.3 | 26.2 | 21.3 |
| Tetralin | - | 14.9 | 0.11 | - |
| Biphenyl | 1.8 | 6.0 | 1.3 | 0.99 |
| Fluorene | 4.6 | 3.0 | 4.5 | 4.7 |
| Phenanthrene/Anthracene | 12.9 | 5.2 | 10.3 | 11.0 |
| Tetrahydrophenanthrene | - | 3.9 | 0.04 | - |
| Octahydrophenanthrene | - | 2.6 | - | - |
| Pyrene | 8.4 | 3.8 | 8.4 | 7.6 |
| Benzofluorene | 3.2 | 1.0 | 1.3 | 1.9 |
| Benzopyrene | 3.3 | 0.69 | 1.1 | 2.0 |
| Total > MW 128 | 71.05 | 76.23 | 33.64 | 52.81 |

From a consideration of the results given in the table it will be seen that catalytic hydrogenation produces

polycyclic saturated species such as tetralin and the tetra and octa hydrophenanthrenes at the expense of their parent unsaturated compounds whilst in the case of thermal hydrogenation without a preceeding catalytic hydrogenation the unsaturated polycyclics survive a thermal hydrogenation reaction substantially unchanged.

The component "Total > MW 128" describes all compounds having molecular weights in excess of 128 including those specifically reported elsewhere in the table and gives a general indication of the concentration of polycyclic compounds present in the feedstock and products. It will be noted that in accordance with the invention the concentration of such compounds exiting the thermal hydrogenation stage is only 33.64% compared with 52.81%.

The slight increase in ">MW128 compounds" upon catalytic hydrogenation is due to the formation of biphenyls which however are readily hydrogenated under thermal hydrogenation conditions, and the production of tetralin at the expense of naphalene.

CLAIMS

1. A process for the production of polynuclear saturated compounds which comprises subjecting a feedstock comprising predominantly unsaturated polynuclear hydrocarbons to catalytic hydrogenation at a temperature of not more than 500°C with a hydrogen-containing gas at a partial pressure of at least 100 bar, the ratio of hydrogen to feedstock being not less than 0.9 $Nm^3$ ($H_2$) per kg (feedstock), said catalyst being one which has substantially no propensity for cracking or for initiating and sustaining gasification reactions at said temperatures and pressures.

2. A process for the production of methane containing gases which process comprises subjecting a feedstock comprising predominantly unsaturated polynuclear hydrocarbon compounds to catalytic hydrogenation at a temperature of not greater than 500°C with a hydrogen containing gas at a partial pressure of at least 100 bar, the ratio of hydrogen to feedstock being not less than 0.9 $Nm^3$ $H_2$ per kg (feedstock), said catalyst having substantially no propensity for cracking or for initiation and sustaining gasification reactions at said temperatures and pressures, to produce a product comprising saturated polynuclear hydrocarbon compounds and thereafter subjecting said product to non-catalytic thermal hydrogenation at a

temperature of not more than 950°C in the presence of a hydrogen-containing gas.

3. An integrated process for the production of substitute natural gas or precursors therefor comprising thermally hydrogenating a hydrocarbon oil in the presence of a hydrogen containing gas at a temperature of not more than 950°C, separating from the reaction product a methane rich gas, unreacted hydrogen-containing gas and a condensible material comprising poly and monocyclic compounds, further separating the condensible material into mono and polycyclic components, reacting the monocyclic components over a reforming catalyst to produce a hydrogen-containing product, reacting a mixture of said hydrogen-containing product and said polycyclic component in a ratio of not less than 0.9 $Nm^3$ of hydrogen-containing product per kg of polycyclic component in the presence of a hydrogenating catalyst having substantially no propensity for cracking or for initiating and sustaining gasification reactions, at a temperature of not more than 500°C and at a hydrogen-partial pressure of at least 100 bar thereby to form a reaction product comprising saturated polynuclear hydrocarbons and thereafter thermally hydrogenating said saturated polynuclear hydrocarbon reaction product together with said hydrocarbon oil.

4. A process as claimed in any one of Claims 1 to 3 in

which said catalytic hydrogenation is affected at a temperature of from 350°C to 450°C.

5. A process as claimed in any one of Claims 1 to 4 in which said catalytic hydrogen is effected at a hydrogen partial pressure of from 100 to 200 bar.

6. A process as claimed in any one of Claims 1 to 5 in which the ratio of hydrogen to said predominently unsaturated polynuclear hydrocarbons is from 1.2 to 2.5 $Nm^3$ ($H_2$) per kg (hydrocarbons).

1/1

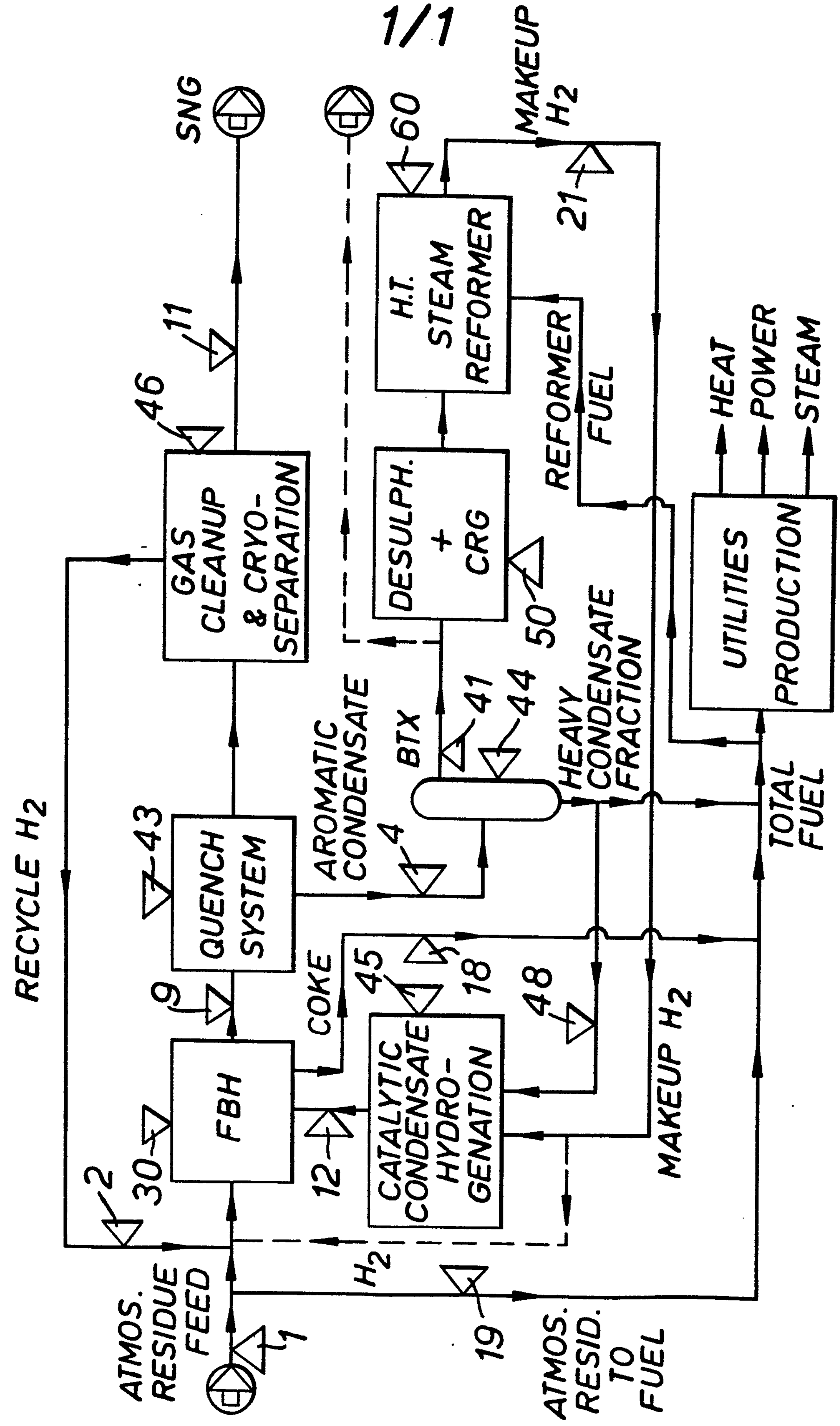

ATMOS. RESIDUE FEED
1
RECYCLE $H_2$
2
30
FBH
9
QUENCH SYSTEM
43
GAS CLEANUP & CRYO-SEPARATION
46
11
SNG
$H_2$
19
ATMOS. RESID. TO FUEL
12
COKE
CATALYTIC CONDENSATE HYDRO-GENATION
45
18
48
AROMATIC CONDENSATE
4
BTX
41
44
HEAVY CONDENSATE FRACTION
DESULPH. + CRG
50
H.T. STEAM REFORMER
60
MAKEUP $H_2$
21
REFORMER FUEL
MAKEUP $H_2$
TOTAL FUEL
UTILITIES PRODUCTION
HEAT
POWER
STEAM

0103948

Application number

European Patent Office

# EUROPEAN SEARCH REPORT

EP 83303887.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB - A - 2 060 684 (LINDE AKTIEN-GESELLSCHAFT)<br>* Claims; page 1, line 3 - page 3, line 29 *<br>-- | 1,4 | C 07 C 9/04<br>C 10 G 45/58<br>C 10 G 69/06<br>C 07 C 4/00<br>C 07 C 5/10 |
| A | GB - A - 2 071 133 (COAL INDUSTRY (PATENTS) LIMITED)<br>* Claims; page 1, lines 5-120 *<br>-- | 1,4 | |
| A | GB - A - 2 093 477 (CHIYODA CHEMICAL ENGINEERING AND CONSTRUCTION CO. LTD.)<br>* Claims; page 1, line 1 - page 8, line 41 *<br>-- | 1,4-6 | |
| | | | TECHNICAL FIELDS SEARCHED (Int. Cl. 3) |
| D,Y | GB - A - 2 062 000 (BRITISH GAS CORPORATION)<br>* Fig. 5; page 2, lines 5-10; example 5; claims 4,5 *<br>-- | 2,3 | C 10 G 47/00<br>C 10 G 45/00<br>C 07 C 9/00<br>C 07 C 4/00<br>C 07 C 5/00<br>C 07 C 13/00<br>C 10 G 69/00 |
| Y | US - A - 4 313 017 (MC. GINNIS)<br>* Column 1, lines 18-22 *<br>-- | 1,3 | |
| A | DE - A - 1 568 180 (BADISCHE ANILIN & SODAFABRIK)<br>* Claim; example 3 *<br>-- | 1,5 | |

The present search report has been drawn up for all claims

| Place of search | Date of completion of the search | Examiner |
|---|---|---|
| VIENNA | 29-11-1983 | STÖCKLMAYER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

0103948

Application number

European Patent Office

# EUROPEAN SEARCH REPORT

EP 83303887.0

| Category | Citation of document with indication, where appropriate, of relevant passages | Relevant to claim | CLASSIFICATION OF THE APPLICATION (Int. Cl.³) |
|---|---|---|---|
| A | US - A - 3 313 859 (DOANE)<br>* Claims 1,6,9 *<br>-- | 1,4,5 | |
| A | GB - A - 1 007 305 (BERGWERKSVERBAND)<br>* Example *<br>-- | 1,4,5 | |
| X | US - A - 3 349 140 (A.W. WEITKAMP)<br>* Example 1; claims 1-6<br>-- | 1 | TECHNICAL FIELDS SEARCHED (Int. Cl.³) |
| X | US - A - 1 894 925 (J. VARGA)<br>* Example *<br>-- | 1,4,5 | |
| A | US - A - 3 732 085 (N.L. CARR et al.)<br>* Column 3, line 30 - column 4, line 2; especially column 3, lines 39,68,71 *<br>---- | 2,3 | |